# EUROPEAN PATENT APPLICATION

(11) **EP 1 147 774 A1**
(43) Date of publication of application: **24.10.2001**
(21) Application number: 00201444.7
(22) Date of filing: 20.04.2000
(51) Int. Cl.: A61K 35/52, A61K 31/522

(54) **Method for improving the quality of sperm for artificial insemination of animals**

(71) Applicant: Stichting Dienst Landbouwkundig Onderzoek, 6708 PD Wageningen (NL)
(72) Inventor: Matthijs-Rijsenbilt, Jacoba Johanna, 8253 BB Dronten (NL); Woelders, Henri, 8212 EA Lelystad (NL)
(74) Representative: Prins, Adrianus Willem

(57) **Abstract**

A method for the artificial insemination of animals, which method comprises artificially inseminating the animal with sperm, combined with a phosphodiesterase inhibitor or a functional equivalent thereof and optionally a soluble salt of an earth alkaline metal and the use of a phosphodiesterase inhibitor and a soluble salt of an earth alkaline metal in a composition which further comprises sperm for the reduction of the recruitment of polymorphonuclear neutrophils and a method for artificial insemination of animals.

## Description

The present invention pertains to a method for improving the artificial insemination of animals as well as the use of the method for improving the quality and survival rate of sperm and reducing the recruitment of Polymorphonuclear Neutrophils (PMN) in the female genital tract.

When breeding animals in an industrial environment it is economically desirable that the animals generally have predetermined or previously estimated qualities. As a consequence thereof, regardless whether it involves animals such as cows whereby breeding is limited at the level of individual animals or for example with pigs at the level of selection lines, there is a continuous need to improve breeding methods for animals both in qualitative aspects as well as in quantitative aspects and to improve the efficiency of such breeding method.

One of these improved methods already known in the art is artificial insemination (AI). With artificial insemination, first an ejaculate is obtained from a male animal. Portions of the ejaculate can be inseminated as such or can be treated to improve its quality or to obtain more doses for insemination. When the sperm is treated, usually the seed is analysed, diluted, preferably in a suitable medium such as a BTS-medium, and further treated using methods known in the art to provide a suitable sample that can be used as an inseminate.

By pretreating and/or diluting the ejaculate it is possible to obtain from one ejaculate a large number of doses which each can be used for insemination. For instance, from one pig ejaculate 20-40 doses suitable for artificial insemination can be obtained. Such a dose generally contains about 2.5 billion sperms. The presently known methods for artificial insemination of pigs result in a fertility result of about 90% and about 11 piglets per litter.

Although this is an acceptable result, it would be desirable to obtain a lower insemination dose without a negative effect to the fertility result and/or litter size. This would lead to more homogeneous populations and to a more uniform product. Such a desirable improvement would lead to a more efficient use of the sperm of animals and hence to substantial economic advantages. Other advantages are an increased genetic gain in nucleus breeding because of larger number of progeny, an increased rate of dissemination of superior traits into the population and an increased efficiency of reproduction (higher litter size, higher farrowing rate) and less sperm needed per insemination (more diluted semen means a cheaper production of semen). The latter advantage could stimulate the use of frozen semen for routine AI, which has important veterinary health advantages (prevention of diseases).

However, one of the disadvantages of insemination whether through natural or artificial routes is that within hours after insemination the number of sperm cells in the female genital tract is reduced dramatically. This is ascribed to phagocytosis of sperm by uterine leukocytes such as Polymorphonuclear Neutrophils (PMN). These PMNs are recruited in vast numbers to the lumen of the uterus shortly after (artificial) insemination. The result is that a large amount of the inseminated sperm is not used. For instance, it has been found that after 8 hours only about 1% of the amount of originally artificially inseminated sperm remains and after 24 hours only 0.1% of the inseminated amount. This is one of the reasons why so many sperm per insemination is needed. Moreover, the rapidly declining sperm numbers also lead to a lower fertility rate and hence is a disadvantage of the known insemination methods. This is even more disadvantageously when the exact time of ovulation is difficult to estimate, which is the case with, for instance, pigs. It has been shown that the negative effect of too early insemination, respective to ovulation, can not be "repaired" by increasing the sperm dosage to as much as 6 billion sperm per dose (Steverink et al, J. Reprod. Fert. 1997, 111, 165-171). More sperm may simply elicit more PMN recruitment and phagocytosis activity.

The combination of increased phagocytosis and the uncertainty of the ovulation lead to an even lower fertility rate. And even if the time of ovulation and consequently the time of insemination can be determined adequately, the insemination at the right time will still be of critical importance for a good fertility rate. This means that the relative fast phagocytosis of the sperm is still a disadvantage of the presently known techniques.

In the field of human in vitro fertilisation techniques there also exists a need for the improvement of fertilisation results. With human in vitro fertilisation techniques it is known that an improved fertilisation result can be obtained by capacitation of the sperm. Capacitation is connected with a large amount of specific cellular changes in the sperm related to the ability to the fertilisation the oocyte. Caffeine can (partly) accelerate certain phases of capacitation. Caffeine stimulates the motility of sperm and aids in inducing the state of so called 'hyperactivated motility', which is seen as a specific stage of capacitation. Due to the increased motility of the sperm the in vitro fertility rate is increased, yet considerably reduces the lifespan of the sperm.

It is stressed that a major disadvantage of the capacitation of seed is that after capacitation of seed the sperm has a shorter lifespan. This reduces the period from the moment of insemination until the moment at which the sperm is no longer sufficiently fertile considerably and hence reduces the time available for fertilisation. This implicates that the use of capacitation techniques on sperm for the use in artificial insemination with animals, preferably mammals such as pigs or birds is not suitable because of the shortened life expectancy of the sperms. Especially when combined with the previously discussed difficulty in determining the time of ovulation of pigs, capacitation of sperm does not appears to lead to the desired improvements.

One of the goals of the present invention is to come to a method and/or a composition which leads to an improved fertility rate. It is also desirable to come to improved methods for artificial insemination and compositions for use in artificial insemination. A further goal is to increase the functional life span of sperm and/or reduce the phagocytosis of sperm and/or reduce the recruitment of leukocytes.

Surprisingly, the present inventors have now found that a treatment of sperm, applied to spermatozoa, for example in vitro, reduces the rate at which sperm is phagocytosed, in particular in vivo. They also found that a comparable treatment, when applied to an inseminate significantly reduces the recruitment of polymorphonuclear leukocytes (synonym: neutrophile granulocytes) in vivo after artificial insemination. The treatment comprises the contacting of the sperm with a phosphodiesterase inhibitor, preferably in combination with a source of a soluble salt of an earth alkaline metal, such as a soluble salt of calcium or magnesium, preferably calcium in a suitable medium. A strongly reduced phagocytosis of sperm is found and at the same time a significant prolongation of the presence of fertilisation competent sperm in the genital tract.

Considering the rate at which phagocytosis normally takes place after insemination and thus the rate at which the number of sperm in the uterus is reduced, a reduction or postponement of phagocytosis as is found with the present invention leads to an improved fertilisation rate and/or to the possibility of using lower insemination doses.

The present invention is therefore characterised by a method for the artificial insemination of animals which method comprises a step wherein the animal is inseminated with a composition which comprises, additionally to the semen, a phosphodiesterase inhibitor or functional equivalent thereof and a soluble salt of an earth alkaline metal, preferably a calcium source such as a soluble calcium salt.

By the method according to the invention it suffices to use a lower dose of sperm for obtaining a similar fertilisation rate. Also a longer interval between insemination and ovulation becomes permissible without affecting the fertilisation rates significantly. The semen is found to have a prolonged fertility. Probably at least partially because phagocytosis is strongly reduced. This will maintain the population of sperm in the uterus at a higher level than is found with conventional insemination. By this the population of sperm in the oviducts is maintained or sustained for a prolonged period of time, thereby strongly increasing the chances of fertilisation.

The invention also pertains to a method for the reduction of the recruitment of leukocytes by the artificial insemination of mammals by the addition of a phosphodiesterase inhibitor or functional equivalent thereof and a calcium salt to the inseminate.

The media used in the art for artificial insemination are generally salt-containing solutions. Examples thereof are Beltsville TS (BTS), Modified Modena (MM), X-cell and Vital (Instruments Médicine Véterinaire, L'Aigle, France), MR-A, Kiev, Beltsville Liquid, Zorlesco, IVT, Modena, Bütschwil, BW25, or Androhep (Weitze, KF, 1991, Long-term storage of extended boar semen. In: Boar Semen Preservation II, L.A. Johnson and D.Rath, Eds. Proceedings of the 2nd International Conference on Boar Semen Preservation; Reproduction in Domestic Animals Supplement 1, 145-164; Johnson-LA; Aalbers-JG; Grooten-HJG. Artificial insemination of swine: Fecundity of boar semen stored in Beltsville TS (BTS), Modified Modena (MM) or MR-A and inseminated on one, three and four days after collection. Zuchthygiene. 1988, 23: 2, 49-55; Johnson-LA; Aalbers-JG Artificial insemination of swine: fertility using several liquid semen diluents. Proceedings of the 8th International Pig Veterinary Society Congress, Belgium, Aug. 27-31, 1984. 1984. University Faculty of Veterinary Medicine; Ghent; Belgium; Johnson-LA; Aalbers-JG; Willems-CMT; Rademaker-JHM; Rexroad-CE Jr. Use of boar spermatozoa for artificial insemination. III. Fecundity of boar spermatozoa stored in Beltsville liquid and Kiev extenders for three days at 18 deg C. Journal-of-Animal-Science. 1982, 54: 1, 132-136; Johnson-LA; Aalbers-JG; Willems-CMT; Rademaker-JHM. Fertility of boar semen stored in BL-1 and Kiev extenders at 18 deg C for three days. Proceedings of the International Pig Veterinary Society 6th Congress, Copenhagen, June 30th-July 3rd, 1980. 1980, 33.

Most of the here-in-before exemplified media are intended for pigs. For bovine animals a tris-citrate-yolk medium is known as well as other media. Other media for other species are well known in the art and it is within the realm of the skilled man to find suitable media for artificial insemination without exercising any inventive experimentation.

Generally, the media do not contain calcium. Moreover, they generally contain chelators like Na₂EDTA and/or sodium citrate. It is thought that these substances bind divalent and trivalent cations and therefore keep the free calcium and magnesium concentrations very low.

The amount of added calcium salt or equivalent thereof is such that the chelators which may optionally be present in conventional or commercial media, are saturated. This means that no or virtually no free chelators are present and thus unchelated calcium is present in the media.

In a preferred embodiment the earth alkaline metal salt is added to the usual insemination media in an amount of up to 100 mmol per litre inseminate, preferably from 0.01 to 50 mmol added earth alkaline metal ions per litre inseminate.

In a preferred embodiment the calcium salt is added to the usual insemination media in an amount of up to 10 mmol per litre inseminates, preferably from 0.1 to 8 mmol added calcium ions per litre inseminate.

In a preferred embodiment the calcium ion source is added to the composition for insemination in an amount of 5 mmol added calcium chloride per litre inseminate with higher preference from 0.1 to 4 mmol added calcium per litre.

The phosphodiesterase inhibitor (PDE) which preferably is used in the composition according to the invention is caffeine. Other preferred phosphodiesterase inhibitors that are suitable for use in the method according to the present invention are other xanthine based compounds such as theophylline, theobromine, isobutylmethylxanthine or papaverine.

Without wishing to be bound to any theory, it is thought that PDEs reduce the decomposition of cAMP (cyclic Adenosine MonoPhosphate). A similar effect is achieved by adding cAMP or by using compounds that stimulate the formation of cAMP or increase the amount of cAMP, for instance by stimulating the enzyme adenylate cyclase (AC stimulator). An examples thereof is dibutyryl cAMP.

Adding cAMP inhibits the phagocytosis of sperm in vitro. Within the boundaries of the present invention, PDEs and cAMP increasing compounds or compositions are considered as functional equivalents. This means that PDEs can be replaced wholly or partially by other compounds or compositions that increase the amount of cAMP present.

In a preferred embodiment, the PDE inhibitor is at least partially replaced by a compound that increases the amount of cAMP.

In a preferred embodiment the caffeine is added to the composition comprising sperm in an amount of up to 10 mmol per litre inseminate, preferably up to 5 mmol per litre with a higher preference in an amount of 0.1 to 3 mmol per litre. The upper limit for the amount of caffeine that can be added is set at 10 mmol per litre; however, when other phosphodiesterase inhibitors are used this upper limit may fluctuate. Depending on the PDE or functional derivative or combination thereof the effective amount may vary widely. It is envisaged that, depending on the activity of the PDE or functional derivative, the amount added to the composition comprising sperm may vary from 0.01, 0.1, 0.5, 1, 2, 3 ,4, 5, 6, 7, 8, 9, 10 mmole per litre inseminate up to 20, 30, 40, 50, 60, 70, 100 mmole per litre inseminate. The preferred ranges can be determined by the skilled man by experimentation such as disclosed in the Examples. Essential in this respect is that the upper limit for the phosphodiesterase inhibitor or functional equivalent thereof is the amount that avoids a mainly unwanted or undesired toxic effect of the phosphodiesterase inhibitor.

In a further aspect of the invention other routes can be used to inhibit phagocytosis of sperm after insemination, apart from using PDE inhibitors or adenylate cyclase stimulants. These alternatives are included in the functional equivalents of the PDEs in the light of the present invention. A preferred route is the masking of the ligands involved in binding of PMN to spermatozoa. It is known that sperm can bind opsonins, e.g. antibodies, that allow the subsequent binding of PMN. Live, intact sperm possess intrinsic ligands that allow binding by PMN. For the latter class of ligands no prior opsonisation is necessary. This allows the immediate start of phagocytosis, right after insemination. These ligands use sugar moieties for the binding with PMN. Therefore, the following two methods are also used to inhibit phagocytosis:
- masking of the ligands, e.g. by adding a complementary carbohydrate binding molecule; and
- enzymatic degradation of the ligand, e.g. by using deglycosylating enzymes or agents.
Masking of the ligands is preferred. The phagocytosis of boar spermatozoa in vitro by PMN is inhibited strongly by adding polysulphated polymers, e.g. glycosaminoglycans like heparin, due to binding of these substances to the plasma membrane and shielding off the ligands involved in binding by PMN.
Another way to inhibit phagocytosis of sperm is to capacitate sperm. It was found that treating the semen for, for example in vitro, capacitation reduced strongly the phagocytosis of sperm by PMN, in particular in vitro. Without wishing to be bound by theory, capacitation is considered as a series of cellular changes, notably changes of the surface of the cell membrane, and involving deglycosylation of membrane proteins and membrane lipids, by which the spermatozoa acquire the ability to penetrate and fertilise the oocyte. The ligands involved in phagocytosis are lost, and the ability to bind serum-derived opsonins is decreased.

The phosphodiesterase inhibitor or functional equivalent thereof can be added at any moment prior to insemination to the composition comprising sperm. Suitable moments are for instance directly after collecting the ejaculate or during the processing of the ejaculate to dosages in a form and amount suitable for artificial insemination. The phosphodiesterase inhibitor, preferably caffeine, can be added to the composition comprising sperm directly prior to the insemination. There is a preference for the addition of the combination of calcium and caffeine immediately prior to insemination. Another preferred embodiment is that a composition comprising calcium and caffeine is applied directly after the insemination with a conventional insemination dosage. This means that first insemination with a composition comprising sperm takes place directly followed by a separate step in which a composition comprising calcium and caffeine are administered. Finally it is also possible to first administer a composition comprising calcium ions and the PDE inhibitor and thereafter carrying out the insemination step.

It has been found and this is a further aspect of the present invention that an increased fertility rate is obtained when first the sperm are administered in a suitable medium directly followed by the administration of a solution containing caffeine and calcium salt in comparison to a similar volume of a solution which contains sperm together with caffeine and the calcium salt.

During in vivo experiments whereby female mammals were inseminated with a dosage of sperm in a conventional medium and subsequently were administered a dose of caffeine and calcium salt in a suitable medium, a lower reduced rate of recruitment of leukocytes and a reduced phagocytosis was found.

Administration of a composition comprising phosphodiesterase inhibitor or functional equivalent thereof and a calcium salt after conventional insemination may for certain animals therefore also lead to an improved fertility rate and is therefore within the scope of the invention.

The storage for several days of the treated inseminate prior to the use thereof generally has a less positive influence on the life span of the sperm. It is therefore preferable to add the phosphodiesterase and calcium ions containing component to the sperm immediately prior to the insemination. It is within the scope of the present invention to provide for the composition with the phosphodiesterase inhibitor or functional equivalent thereof and the calcium ions in a separate holder. The composition comprising the sperm can be provided in another separate holder. These two compositions can be mixed directly prior to insemination. Accordingly, the invention in a further aspect relates to a kit comprises a holder with the phosphodiesterase inhibitor or functional equivalent thereof and calcium and in a preferred embodiment to the combination of the holder with the phosphodiesterase inhibitor or functional equivalent thereof and calcium and the other holder with the composition comprising sperm.

The method according to the invention is principally suitable for all types of sperm and is thus applicable to all types of animals including humans, preferably mammals and birds, more preferably pigs. By applying the method according to the invention to pig sperm, the amount of sperm and inseminate can considerably be reduced without lowering the fertility rate. The method according to the invention accordingly allows to obtain a larger amount of sperm doses from one ejaculate which promotes the desirable homogeneity of the population. Likewise, it is possible to use a conventional amount of sperm in the inseminate in a method according to the invention to obtain an improved fertility rate.

The invention will be further exemplified by means of the following examples, but by no means is limited thereto.

### Example I:

### The influence of caffeine and caffeine plus Ca²⁺ on phagocytosis of sperm by PMN in vitro

### MATERIALS AND METHODS.

### Media.

Tyrode's medium was used as described as "standard Tyrode's medium" in Harkema *et al*. (Harkema, W, Harrison, RAP, Miller, NGA, Topper, EK, and Woelders, H ,1998, Enhanced Binding of Zona Pellucida Proteins to the Acrosomal Region of Intact Boar Spermatozoa in Response to Fertilizing Conditions: A Flow Cytometric Study. Biology of Reproduction, 58, 421-430), but without propidium iodide, and lacking BSA, but supplemented with 15% (v/v) sow serum treated to heat-inactivate complement. Serum was prepared from blood collected from 11 primiparous sows, pooled, and frozen in aliquots at -80 °C.

### Isolation and Preparation of PMN.

Per experiment, 20 ml peripheral blood of a single Dutch Landrace sow (each experiment a different sow) was collected in heparinised vacutainers (Venoject, Omnilabo, Breda, The Netherlands), and subsequently diluted with an equal volume of phosphate buffered saline (PBS). A number of 15-ml screw-capped polypropylene centrifuge tubes were each filled with 3 ml Ficoll Paque (Pharmacia, Biotech Benelux, Roosendaal, The Netherlands). Per tube, 4 ml of diluted blood was carefully layered over the Ficoll and centrifuged at 400 x g for 35 min. The supernatant, including the mononuclear cells at the interface, was removed. The pellet was resuspended in 2 ml of ice-cold distilled water to lyse the erythrocytes. Isotonicity was restored after 45 s by addition of 1 ml of 27 g l⁻¹ NaCl. The PMN were pelleted by centrifugation (10 min at 400 x g) and the lysis procedure was repeated. After that, each PMN pellet was washed two times more in 5 ml PBS, and finally resuspended in 1 ml PBS. Subsequently, all cell suspensions were pooled together, mixed well, and subjected to centrifugation. Finally, the PMN were resuspended in TM-s with inactivated complement, and the cell concentration was determined using a haemocytometer. The PMN preparation was stored overnight at 4 °C. Just prior to use, the PMN suspension was mixed, and centrifuged at 400 x g for 10 min. The PMN were resuspended in TM-s with inactivated complement and adjusted to 10 x 10⁶ cells ml⁻¹. The above procedure for PMN isolation yielded a preparation containing more than 90% granulocytes, of which about 85% were neutrophils. The viability, assessed by trypan blue exclusion was > 98%.

### Preparation of Semen Samples.

Semen was obtained from Dutch A.I. stations. Per experiment, semen was used from a single Yorkshire breeding boar (each experiment a different boar). At the AI-station, the semen was diluted to a concentration of approximately 30 x 10⁶ cells/ml in Beltsville Thawing Solution (BTS) (Johnson *et al*., 1988). The diluted semen was stored at 17 °C for up to 48 hours, until use.

The diluted semen in BTS was stained at room temperature with 10 µmol l⁻¹ of the DNA-binding fluorescent dye Hoechst 33342 (Sigma, Brunschwig chemie, Amsterdam, The Netherlands) for at least 30 min. Subsequently, 3 ml of the semen was washed by centrifugation through two layers of 35 and 70% (v/v) Percoll (Sigma) in saline respectively (Harrison *et al*., 1993). The pelleted sperm were resuspended in TM and the concentration was adjusted to 20 x 10⁶ cells ml⁻¹ with TM using a haemocytometer.

### Phagocytosis Assay.

Aliquots of 80 µl of the PMN suspension (see PMN isolation and preparation) in TM or TM-s with either intact or inactivated complement were transferred to a 96-well polystyrene microtest plate. To each well, 20 µl of sperm suspension (see preparation of semen samples) was added and the test plate was placed in a humidified incubator at 38 °C, with 5% CO₂ in air. The final concentrations of PMN, sperm, and serum were 8 x 10⁶ ml⁻¹, 4 x 10⁶ ml⁻¹, and 12% (v/v), respectively. The samples were then incubated, while being gently swirled on a test plate shaker. After 15, 30, 45, 60 or 90 min, samples were transferred quantitatively into tubes containing an equal volume of 40 mg ml⁻¹ heparin (Sigma) in PBS. The heparin facilitates dissociation of agglutinated PMN. The samples were mixed very well, left for 15 min, and mixed again. Then, subsamples of 75 µl were fixed by addition of 25 µ 1 of 2% (v/v) glutaraldehyde (Fluka, Brunschwig chemie, Amsterdam, The Netherlands) in PBS. 'Blank' samples, i.e. sperm without PMN (80 µl TM-s mixed with 20 µl sperm suspension), were incubated in parallel in order to monitor sperm survival during the treatment. Also 'reference' samples, i.e. frozen-thawed (killed) semen, were incubated in parallel with the PMN to have a reference for the phagocytotic activity of the PMN. This frozen-thawed semen was taken from a large stock of semen from one ejaculate, and was washed 3 times in PBS prior to use.

### Microscopical Evaluation of Phagocytosis and Sperm Viability.

For evaluation of phagocytosis, wet mounts of the fixed samples were examined using a combination of phase-contrast and fluorescence microscopy (Olympus BH2, Tokyo, Japan) at 400x magnification. These mounts enabled us to observe and count fluorescently labelled spermatozoa inside and outside the phagocytes. By focusing at different levels in the mount, spermatozoa that were located above or below the PMN could be clearly distinguished from those that were phagocytosed. Moreover, in many PMN that had ingested a spermatozoon the presence of the sperm nucleus or tail caused a conspicuous change of the shape of the PMN. Two hundred spermatozoa were evaluated and classified as in- or outside the PMN (phagocytosed or not). The same fixed mounts were used to assess the acrosome morphology of the non-phagocytosed spermatozoa at the moment of fixation. One hundred cells were classified, using phase-contrast microscopy at 400x magnification, as NAR (normal apical ridge), NAR' (slightly altered normal apical ridge), DAR (damaged apical ridge), MAR (missing apical ridge) and LAC (loose acrosomal cap), as described by Pursel *et al*. (Pursel VG, Schulman LL and Johnson LA, 1978, Distribution and morphology of fresh and frozen-thawed sperm in the reproductive tract of gilts after insemination *Biology of Reproduction* **19** 69-76.).

### RESULTS

### Experiment 1.

In this experiment the semen had been stored for 48 hours in BTS at 17 °C.

### In vitro phagocytosis of sperm by PMN

### I. Without pre-incubation

| | % of phagocytosed sperm | | |
|---|---|---|---|
| Control | 64 | 78 | 71 |
| Caffeine 1 mM | 20 | 22 | 18 |

### II. After pre-incubation

| | % of phagocytosed sperm | | | |
|---|---|---|---|---|
| Phagocytosis time | 15 min | 30 min | 60 min | 90 min |
| Control | 62 | 60 | 70 | 69 |
| Control pre-inc* | 49 | 55 | 68 | 71 |
| Db-cAMP¹0.1 mM pre-inc* | 35 | 59 | 60 | 65 |
| Caffeine 2 mM pre-inc* | 22 | 15 | 29 | 20 |
| Db cAMP 0.2 mM pre-inc* | 47 | 47 | 58 | 54 |

| | | | | |
|---|---|---|---|---|
| ¹ Db-cAMP = dibutyryl-cAMP | | | | |
| * Preincubated during 30 min at 38 °C | | | | |

*Sperm viability: After adding active compound and pre-incubation at 38°C:*

| | % motile sperm | %NAR-DAR-MAR-LAC |
|---|---|---|
| Control | 60 | 68-23-01-08 |
| Control pre-inc.* | 60 | 61-32-00-07 |
| Db-cAMP pre-inc* | 60 | 67-23-02-08 |
| Caffeine pre-inc* | 60 | 53-30-02-15 |

| | | |
|---|---|---|
| * Preincubated during 30 min at 38 °C | | |

*Sperm viability: In blank samples, i.e. after adding the active compound, after pre-incubation at 38 °C, and measured during incubation parallel to the phagocytosis incubation, but without PMN.*

| | % NAR-MAR-MAR-LAC | |
|---|---|---|
| Phagocytosis time | 30 min | 60 min |
| Control | 56-35-00-09 | |
| Controle pre-inc.* | 56-34-00-10 | |
| Db cAMP pre-inc* | 56-36-02-06 | |
| Caffeine pre-inc* | 37-39-03-21 | |
| Db cAMP (2x) | 54-36-02-08 | |
| Control | | 44-40-04-12 |
| Caffeine | | 37-36-05-22 |

| | | |
|---|---|---|
| * Preincubated during 30 min at 38 °C | | |

### Experiment 2

### Phagocytosis of sperm; mean of duplicates

### I. Without pre-ïncubation:

| Time (min) | 15 | 30 | 60 | 15 | 30 | 60 |
|---|---|---|---|---|---|---|
| | % phagoc. sperm | | | %inhibition* | | |
| 0 mM caffeïne | 53 | 76 | 77 | - | - | - |
| 0.2 mM caffeïne | 48 | 73 | 75 | 9 4 3 | | |
| 0.5 mM caffeïne | 40 | 67 | 68 | 25 | 12 | 12 |
| 1 mM caffeïne | 29 | 58 | 62 | 45 | 24 | 19 |
| 2 mM caffeïne | 28 | 51 | 56 | 47 | 33 | 27 |

### II. After pre-ïncubation:

| Time (min) | 15 | 30 | 60 | 15 | 30 | 60 |
|---|---|---|---|---|---|---|
| | % phag. Sperm | | %inhibition* | | | |
| 0 mM caffeine | 47 | 64 | 63 | - | - | - |
| 0.5 mM caffeine | 29 | 28 | 42 | 38 | 56 | 33 |
| 1 mM caffeïne | 14 | 26 | 24 | 70 | 59 | 62 |
| 2 mM caffeïne | 9 | 17 | 21 | 81 | 73 | 67 |

| | | | | | | |
|---|---|---|---|---|---|---|
| * Percentage decrease of phagocytosis relative to control | | | | | | |

*Sperm viability: After adding active compound:*

### I. Without pre-incubation:

| caffeine | %motile sperm | %NAR-DAR-MAR-LAC |
|---|---|---|
| 0 mM | 80 | 85-10-01-04 |
| 0.2 mM | 80 | 84-12-01-03 |
| 0.5 mM | 80 | 84-12-02-02 |
| 1 mM | 80 | 87-09-01-03 |
| 2 mM | 80 | 82-13-01-04 |

### II. After pre-incubation:

| | % motile sperm | %NAR-DAR-MAR-LAC |
|---|---|---|
| 0 mM | 80 | 82-14-01-04 |
| 0.5 mM | 80 | 83-14-01-02 |
| 1 mM | 80 | 77-16-01-06 |
| 2 mM | 80 | 73-20-02-05 |

*Sperm viability: In blank samples, i.e. after adding the active compound, after preincubation at 38 °C (when used), and measured during incubation parallel to the phagocytosis incubation, but without PMN.*
Phagocytosis time 30 min.

### I. Without pre-incubation:

| Caffeine | |
|---|---|
| 0 mM | 83-13-00-04 |
| 0.2 mM | 85-12-00-03 |
| 0.5 mM | 87-10-00-03 |
| 1 mM | 85-10-00-05 |
| 2 mM | 79-12-01-08 |

### II. After pre-incubation:

| Caffeine | |
|---|---|
| 0 mM | 85-11-00-04 |
| 0.5 mM | 85-12-01-02 |
| 1 mM | 72-21-01-06 |
| 2 mM | 70-24-01-05 |

### Experiment 3

*Phagocytosis of sperm; mean of duplicates after pre-incubation of sperm for 30 min at 38 °C with and without 1 mM caffeine:*

| Time (min) | 15 | 30 | 60 | 15 | 30 | 60 |
|---|---|---|---|---|---|---|
| | % phag. Sperm | | %inhibition* | | | |
| 0 mM caffeïne | 56 | 71 | 79 | - | - | - |
| 1 mM caffeïne | 32 | 45 | 56 | 43 | 37 | 29 |
| Reference | 51 | 67 | 76 | | | |

| | | | | | | |
|---|---|---|---|---|---|---|
| * Percentage decrease of phagocytosis relative to control | | | | | | |

*Sperm viability: After pre-incubation of sperm for 30 min at 38 °C with and without 1 mM caffeine:*

| | %motile sperm | %NAR-DAR-MAR-LAC |
|---|---|---|
| 0 mM caffeine | 75 | 74-14-02-10 |
| 1 mM caffeine | 65 | 67-24-01-08 |
| reference | 75 | 78-16-02-04 |

*Sperm viability: In blank samples, i.e. after adding the active compound, after preincubation at 38 °C and measured during incubation parallel to the phagocytosis incubation, but without PMN.*

| Phagocytosis time 30 min. | |
|---|---|
| | %NAR-DAR-MAR-LAC |
| 0 mM caffeine | 73-21-01-05 |
| 1 caffeine | 66-24-02-08 |
| Reference | 72-24-01-04 |

### Experiment 4.

Inhibition of phagocytosis of sperm by addition of caffeine or caffeine plus Ca²⁺ to the semen extender, the day before use.
In this experiment it is shown that addition of caffeine is possible directly after collection of the semen. Moreover the augmentation of the effect of caffeine by supplying calcium is shown.
The semen was extended with either regular BTS, or with BTS plus 6 mM of CaCl₂. The semen also received 0, 0.2 or 1 mM caffeine. The semen was then stored for 24 hours at 17 °C. Pre-incubation at 38 °C before the challenge with the PMN was still used to mimic the situation after insemination of the semen into the sow.
The outcome is that the caffeine after one day inhibits phagocytosis.

### % Phagocytosed sperm (mean of duplicates):

### I Without pre-incubation

| Time (min) | 15 | 30 | 60 | 15 | 30 | 60 |
|---|---|---|---|---|---|---|
| | % phag. Sperm | | | %inhibition* | | |
| Regular BTS | 68 | 76 | 82 | - | - | - |
| BTS+0.2mM Caf | 70 | 81 | 75 | - | - | - |
| BTS+1mM Caf | 58 | 59 | 67 | 17 | 22 | 19 |
| BTS/Ca¹ | 64 | 71 | 78 | 7 | 7 | 5 |
| BTS/Ca+0.2mM Caf | 63 | 73 | 76 | 9 | 5 | 7 |
| BTS/Ca+1mM Caf | 53 | 53 | 63 | 23 | 31 | 24 |

### II After pre-incubation for 30 min. at 38 °C

| Time (min) | 15 | 30 | 60 | 15 | 30 | 60 |
|---|---|---|---|---|---|---|
| | % phag. Sperm | | | %inhibition* | | |
| Regular BTS | 67 | 62 | 66 | - | - | - |
| BTS+0.2mM Caf | 59 | 52 | 68 | 13 | 16 | - |
| BTS+1mM Caf | 51 | 42 | 53 | 23 | 33 | 20 |
| BTS/Ca¹ | 66 | 66 | 64 | 2 - 3 | | |
| BTS/Ca+0.2mM Caf | 61 | 59 | 59 | 10 | 5 | 11 |
| BTS/Ca+1mM Caf | 33 | 34 | 57 | 5O | 54 | 14 |

| | | | | | | |
|---|---|---|---|---|---|---|
| * Percentage decrease of phagocytosis relative to control | | | | | | |
| ¹ BTS plus 6 mM CaCl₂ | | | | | | |

### Viability of the sperm after 24 hour storage and after pre-incubation (when used):

### I. Without pre-incubation:

| | %motile sperm | %NAR-DAR-MAR-LAC |
|---|---|---|
| Regular BTS | 75 | 68-27-00-05 |
| BTS+0.2mM Caf | 75 | 69-27-00-04 |
| BTS+1mM Caf | 70 | 58-38-00-04 |
| BTS/Ca¹ | 70 | 65-29-01-05 |
| BTS/Ca+0.2mM Caf | 70 | 59-35-01-04 |
| BTS/Ca+1mM Caf | 70 | 59-39-00-03 |

### II. After pre-incubation:

| | %motile sperm | %NAR-DAR-MAR-LAC |
|---|---|---|
| Regular BTS | 75 | 69-27-01-03 |
| BTS+0.2mM Caf | 75 | 75-22-00-03 |
| BTS+1mM Caf | 70 | 72-23-00-05 |
| BTS/Ca¹ | 65 | 69-26-00-05 |
| BTS/Ca+0.2mM Caf | 65 | 70-25-00-05 |
| BTS/Ca+1mM Caf | 65 | 65-25-01-09 |

| | | |
|---|---|---|
| ¹ BTS plus 6 mM CaCl₂ | | |

*Sperm viability: In blank samples, i.e. measured during incubation parallel to the phagocytosis incubation, but without PMN.*

### I. Without pre-incubation:

| Phagocytosis time: 30 min. | |
|---|---|
| | %NAR-DAR-MAR-LAC |
| Regular BTS | 69-22-00-09 |
| BTS+0.2mM Caf | 73-19-01-07 |
| BTS+1mM Caf | 71-17-01-11 |
| BTS/Ca¹ | 70-17-01-12 |
| BTS/Ca+0.2mM Caf | 65-21-01-13 |
| BTS/Ca+1mM Caf | 57-29-01-13 |

### II. After pre-incubation:

| Phagocytosis time: 30 min. | |
|---|---|
| | %NAR-DAR-MAR-LAC |
| Regular BTS | 64-19-03-14 |
| BTS+0.2mM Caf | 58-26-01-14 |
| BTS+1mM Caf | 43-34-01-22 |
| BTS/Ca¹ | 63-23-00-14 |
| BTS/Ca+0.2mM Caf | 66-21-00-14 |
| BTS/Ca+1mM Caf | 48-33-00-19 |

| | |
|---|---|
| ¹ BTS plus 6 mM CaCl₂ | |

### Experiment 5.

In this experiment the semen was stored for 24 hours at 17 °C in regular BTS plus CaCl₂, but without caffeine. Then, prior to the phagocytosis assay it was mixed with an equal volume of BTS plus 6 mM CaCl₂, and with Caffeine
This experiment showed that addition of 1 mM caffeine reduces the phagocytosis of sperm by PMN in vitro, without adversely affecting sperm viability. The effect was augmented by the presence of calcium ions and by pre-incubation at 38 °C during 30 minutes.

### % Phagocytosed sperm (mean of duplicates):

### I Without pre-incubation

| Time (min) | 15 | 30 | 60 | 15 | 30 | 60 |
|---|---|---|---|---|---|---|
| | % phag. Sperm | | | %inhibition* | | |
| Regular BTS | 68 | 73 | 76 | | | |
| BTS+0.2mM Caf | 70 | 72 | 76 | - | 1 | - |
| BTS+1mM Caf | 62 | 59 | 65 | 9 | 19 | 14 |
| BTS/Ca¹ | 65 | 68 | 78 | 4 7 | | - |
| BTS/Ca+0.2mM Caf | 66 | 63 | 75 | 3 | 14 | 1 |
| BTS/Ca+1mM Caf | 46 | 43 | 66 | 37 | 41 | 13 |

### II After pre-incubation for 30 min. at 38 °C

| Time (min) | 15 | 30 | 60 | 15 | 30 | 60 |
|---|---|---|---|---|---|---|
| | % phag. Sperm | | | %inhibition* | | |
| Regular BTS | 57 | 64 | 75 | - | - | - |
| BTS+0.2mM Caf | 55 | 63 | 59 | 3 | 2 | 29 |
| BTS+1mM Caf | 34 | 41 | 35 | 40 | 37 | 53 |
| BTS/Ca¹ | 51 | 55 | 67 | 10 | 14 | 11 |
| BTS/Ca+0.2mM Caf | 58 | 54 | 64 | - | 16 | 15 |
| BTS/Ca+1mM Caf | 21 | 19 | 31 | 64 | 71 | 59 |

| | | | | | | |
|---|---|---|---|---|---|---|
| * Percentage decrease of phagocytosis relative to control | | | | | | |
| ¹ BTS plus 6 mM CaCl₂ | | | | | | |

*Viability of the sperm after 24 hour storage in regular BTS, followed by mixing with equal volume of regular or modified BTS, and 30 min pre-incubation at 38 °C (when used):*

### I. Without pre-incubation:

| | %motile sperm | %NAR-DAR-MAR-LAC |
|---|---|---|
| Regular BTS | 75 | 70-27-00-03 |
| BTS+0.2mM Caf | 75 | 76-19-01-04 |
| BTS+1mM Caf | 75 | 64-28-01-07 |
| BTS/Ca¹ | 75 | 72-22-00-06 |
| BTS/Ca+0.2mM Caf | 75 | 63-33-01-03 |
| BTS/Ca+1mM Caf | 70 | 65-27-01-07 |

### II. After pre-incubation:

| | %motile sperm | %NAR-DAR-MAR-LAC |
|---|---|---|
| Regular BTS | 70 | 71-23-01-05 |
| BTS+0.2mM Caf | 75 | 66-30-01-03 |
| BTS+1mM Caf | 65 | 69-24-00-07 |
| BTS/Ca¹ | 60 | 70-25-00-05 |
| BTS/Ca+0.2mM Caf | 65 | 67-26-01-06 |
| BTS/Ca+1mM Caf | 60 | 70-24-01-05 |

| | | |
|---|---|---|
| ¹ BTS plus 6 mM CaCl₂ | | |

*Sperm viability: In blank samples, i.e. measured during incubation parallel to the phagocytosis incubation, but without PMN.*

### I. Without pre-incubation:

| Phagocytosis time: 30 min. | |
|---|---|
| | %NAR-DAR-MAR-LAC |
| Regular BTS | 62-28-01-09 |
| BTS+0.2mM Caf | 70-13-01-16 |
| BTS+1mM Caf | 57-21-00-22 |
| BTS/Ca¹ | 69-17-00-14 |
| BTS/Ca+0.2mM Caf | 67-22-00-11 |
| BTS/Ca+1mM Caf | 66-22-01-12 |

### II. After pre-incubation:

| Phagocytosis time: 30 min. | |
|---|---|
| | %NAR-DAR-MAR-LAC |
| Regular BTS | 65-17-00-17 |
| BTS+0.2mM Caf | 70-15-01-14 |
| BTS+1mM Caf | 62-18-00-20 |
| BTS/Ca¹ | 70-18-00-12 |
| BTS/Ca+0.2mM Caf | 67-18-00-15 |
| BTS/Ca+1mM Caf | 66-22-01-11 |

| | |
|---|---|
| ¹ BTS plus 6 mM CaCl₂ | |

### Example II

**The effect of phosphodiesterase inhibitors, CaCl**_{**2**} **and sodium EDTA on sperm phagocytosis by PMN.**

### MATERIALS AND METHODS

### Experimental design:

Oestrus sows were inseminated using H33342 labelled semen with either
(1) 1 billion sperm in 80 ml BTS (reference),
(2) 1 billion sperm in 40 ml BTS succeeded by 2.3 mM caffeine in 40 ml BTS in which the sodium EDTA was replaced by 6 mM CaCl₂ (BTS-Caf/Ca) or
(3) 1 billion sperm in 80 ml BTS with 25 mM sodium EDTA instead of 3.4 mM (BTS-EDTA).

Within an experiment day, there were two sows per treatment. The experiment days were replicated three times. Thus, in all there were six sows per treatment. Per experiment day, one single ejaculate of semen was used. Boar A was used for one experiment day and boar B was used for two experiment days.

### Oestrus synchronization and detection.

Per experiment day, 8 Multiparous Dutch Landrace x Yorkshire sows (parity ranging from 3-12) were purchased from a Dutch breeding farm. At the day of weaning they were transported to and individually housed at a mechanically ventilated pig facility at the property of the Institute for Animal Health and Science in Lelystad. After 4 days, 26-27 hours before insemination, each sow received an intramuscular injection of 750 IU HCG (AUV, Cuijk) for synchronisation of ovulation. At the early morning of the day of insemination, about 15 hours before the expected ovulation, oestrus detection was performed by allowing the sows consecutively to contact a mature boar in his pen. Per experiment day, 6 sows that exhibited a distinct standing heat reflex when mounted by the boar were assigned selected for the experiment and randomly assigned to the three treatment groups..

### Preparation of the semen.

At the day before insemination, semen was collected from a Yorkshire breeding boar with proven fertility at the AI station in Bunnik, and the initial sperm concentration assessed by measuring light scattering at 550 nm. An aliquot of about 8 billion spermatozoa was diluted with an equal volume of BTS (Johnson LA, Aalbers JG and Grooten HJG (1988) Artificial insemination of swine: fecundity of boar semen stored in beltsville TS (BTS), modified modena (MM), or MR-A and inseminated on one, three and four days after collection *Zuchthygiene* **23** 49-55) and then transported in a refrigerated box at 17°C to the institute in Lelystad. In a 17°C room, 3 aliquots of semen with a volume corresponding to2 billion sperm were diluted either with BTS to a volume of 80 or 160 ml, or with modified BTS containing 25 mM sodium EDTA instead of 3.36 mM (BTS-EDTA) to a volume of 160 ml (final EDTA concentration was approximately 23 mM). The three semen preparations were then stained by addition of the membrane-permeable DNA-binding fluorescent dye Hoechst 33342 (Sigma, Brunswich chemie, Amsterdam) to a concentration of 10 µM. The preparations were covered and stored overnight at 17°C. The next day, small samples were withdrawn for evaluation of viability and to determine the exact sperm concentration. Then, the three sperm preparations were each divided equally over two insemination flasks. All semen was transported in an isolated box to the pig facility, combined with two insemination flasks each containing 40 ml of a solution of 2.3 mM caffeine (Sigma) in modified BTS, in which the sodium EDTA was omitted and which contained 6 mM calcium chloride (BTS-Caf/Ca).

### Evaluation of sperm viability.

The percentage of motile spermatozoa was estimated at 38°C, using phase-contrast microscopy at 100x magnification. Acrosome morphology was assessed after fixation with glutaraldehyde (Fluka, Brunswich chemie, Amsterdam), using phase-contrast microscopy at 400x magnification. 2 x 100 spermatozoa were classified as described by Pursel *et al*. (Pursel VG, Schulman LL and Johnson LA, 1978, Distribution and morphology of fresh and frozen-thawed sperm in the reproductive tract of gilts after insemination *Biology of Reproduction* **19** 69-76.).
Prior to the viability evaluations, the sample from the preparation of 2 billion of sperm per 80 ml were mixed 1:1 with BTS-Caf/Ca.

Until this point, the sperm concentration, or the number of sperm per insemination, had been based on the value of the semen sperm concentration provided by the AI station, which was based on the measurement of light-scattering. To determine the sperm concentration more accurately a small sample of each semen preparation was diluted to a final concentration of 0.7 x 10⁶ sperm/ml in 0.1 M sodium citrate, and the number of sperm was determined in a heamocytometer.

### Procedures at insemination and slaughtering.

After oestrus detection (see above), the sows were successively inseminated at intervals of 35 min with one of the six inseminate preparations (see above). The insemination with 1 billion sperm in 40 ml BTS was immediately succeeded by an insemination with 40 ml of BTS-Caf/Ca. The sows were inseminated through the cervix, using a spiral tip catheter (Nifa instruments, Leeuwarden, The Netherlands) lubricated with a sterile bacteriostatic jelly (Johnson&Johnson, Nifa instruments, Leeuwarden). During insemination, back pressure was applied on the sows. Back flow of semen during, or directly after insemination was collected in a stoma bag (Combihesive, Convatec, Woerden). Subsequently, the stoma bag was clipped onto a ring that was fixed around the vulva of the sow with industrial cyanoacrylate glue and secured with tape. When a sow produced much back flow or had urinated into the stoma bag, the bag was replaced by a new one. The content of each stoma bag was emptied in an urine beaker, weighed on a balance (± 0.05 g) and transported on ice to the laboratory for further processing.

The sows were slaughtered at 4 hours after insemination. For slaughter, a sow was moved to an adjacent room and was immediately stunned by intravenous injection of 10 ml of T61 (AUV, Cuijk) and then exsanguinated from the neck artery. After the abdomen was opened, clamps were placed at the most caudal part of the isthmus, at the junctions between uterine corpus and right horn, between cervix and corpus, between vagina and cervix, and at the caudal end of the vagina. The uterus and oviducts were dissected from their ligaments and the genital tract, with the exception of the vulva, was removed as quickly as possible and transported on ice to the laboratory.

### Preparation of the samples.

Bottles with PBS for rinsing were placed on ice. Oviducts, right uterus horn, left uterus horn plus corpus, cervix, and vagina were separated. The vagina was cut longitudinally, placed inside up in a glass dish and was massaged repeatedly in 50 ml PBS.The cervix was treated likewise in 100 ml PBS. The uterus horns were flushed by introducing 35 ml of PBS which was repeately passed from one side to the other and vice versa. These procedures were repeated one, two and three times for cervix, uterus horns and vagina ,respectively.The parts of the genital tract were weighed and were then ground in a meat mill. Then, from each tissue, a 40 g aliquot was diluted twice with PBS and then homogenised in a Sorvall mixer (Meyvis&Co, Bergen op Zoom) during 5 minutes at maximum speed. Subsamples of ± 10 g were stored at-20°C. The right and left oviducts, which had not been flushed, were homogenised together..

Per part of the tractus therecovered rinse fluid was pooled, the total volume was determined, and after thorough mixing, a 10 ml aliquot was fixed by addition of 100 µ 1 of 50% v/v glutaraldehyde (Fluka, Brunswich chemie, Amsterdam). The collected back flow (see procedures at insemination) was handled accordingly. Rinse fluid of vagina and cervix was frequently contaminated with blood. When this was the case, prior to fixation, the 10-ml sample was centrifuged and the pellet was resuspended in 2 ml of ice-cold distilled water to lyse the erythrocytes. Isotonicity was restored after 1 min by addition of 1 ml of 27 g/l NaCl in water. All fixed samples were stored at 4° C.

### Microscopical evaluation of the samples.

Rinse fluid and semen back flow:

Samples were mixed very well prior to use. Numbers of either PMN or sperm, in and outside the PMN, were determined by counting cells in a KOVA-slide heamocytometer (Instruchemie BV, Hilversum), using a combination of phase-contrast and epifluorescence microscopy at 200x magnification. Cells were counted in a part of the grid corresponding to a volume of 0.444µl. In order to make counting easy and reliable the concentration of cells in the samples was sometimes ajusted by dilution with PBS, or by centrifugation, so that the sample would contain between 56 and 225 cells per µl. In case of a still too low cell concentration the cells in an entire grid, or several entire grids were counted, corresponding to a volume of up to 3.6µl of sample. The phase-contrast microscopy enabled us to distinguish the neutrophils from other leukocytes without differential staining. Occasionally May-Grünwald stained smears of pellets, obtained after centrifugation of rinse fluid before fixation, were evaluated to check for the relative amounts of the various leukocytes in the sample. Using combined phase-contrast and fluorescence microscopy, the phagocytosed fluorescent sperm nuclei inside the PMN could be clearly distinguished. To determine the percentage of phagocytosed sperm 2 x 100 cells were assessed in wet mounts at 200-400x magnification, and classified as in- or outside the PMN (phagocytosed or not). Tissue homogenates: After thawing, the samples were mixed very well. A small amount of the homogenate was placed on top of a Bürker Türk haemocytometer and then spread under the cover-slip. The number of spermatozoa was determined within several entire 0.9 µl grids, using combined phase-contrast and fluorescence microscopy at 400x magnification. Cells were counted in four counting chambers (vagina, cervix and uterus horns) or 12-16 counting chambers (oviducts) per sample. By focussing at different levels in the haemocytometer, all the fluorescent sperm in the homogenised tissue slush could be revealed.

### RESULTS

### General

One of the animals inseminated with caffeine was found to have endometritis. The data of that animal were discarded. Inspection of the ovaries showed that six (out of 18) animals had ovulated, or were in the process of ovulation, whereas the others had not yet ovulated. The number of PMN or sperm recovered from these animals did not seem to be affected by the fact whether or not the sows had ovulated at the time of slaughter.

Before insemination, the sperm had a good viability (Table 1). No differences were observed between the semen of the two boars used. After mixing of semen with BTS-Caf/Ca, most of the spermatozoa displayed a more continuous motility.

**Table 1.**

| Viability of sperm in the semen preparations after storage overnight (n=3) | | | |
|---|---|---|---|
| Inseminate | % Motile sperm | % NAR¹ | % LAC² |
| 1. BTS (reference) | 74 (± 5) | 74.3 (± 4.5) | 4.7 (± 1.5) |
| 2. Sperm in BTS, mixed with an equal volume of BTS-Caf/Ca | 75 (± 1) | 75.6 (± 1.5) | 5.3 (± 1.2) |
| 3. BTS + excess EDTA | 67 (±13) | 78.7 (± 1.2) | 4.3 (± 1.2) |

| | | | |
|---|---|---|---|
| Means (± Standard Deviation) of the 3 ejaculates used observations. ¹ Sperm with a normal apical ridge. | | | |
| ² Sperm with a loose or lost acrosomal cap. | | | |

Most of the semen back flow (70-80%) was collected within the first hour after insemination. After insemination with sperm in regular BTS, the volume of the recovered liquid, without urine contamination, varied from 60 to 120% of the inseminated volume. Less variation was observed after insemination with sperm in BTS and BTS-Caf/Ca, whereas considerably more variation was observed after insemination with sperm in BTS-EDTA.

It was achieved to distinguish the neutrophils from other leukocytes in wet mounts by phase-contrast microscopy. We found a good correlation with differential stained smears according to May-Grünwald/Giemsa. More than 95% of the leukocytes in the back flow and in the rinse fluid of the uterus were neutrophils. The percentage of neutrophils in the back flow was not influenced by the composition of the inseminate or by whether or not ovulation had occurred at the time of slaughter. In the cervix and vagina the proportion of neutrophils had more variation, and was somewhat lower (70-90%), especially in sows inseminated with sperm in BTS + BTS-Caf/Ca (about 40%).

The brightly fluorescent nuclei of the H33342-stained spermatozoa could be clearly observed inside the phagocyte and in the tissue homogenates.

### PMN recruitment.

The numbers of total recovered PMN per sow differed markedly between the individual animals. PMN recruitment was not correlated with age or parity of the sow. The total numbers of PMN did not differ significantly between the inseminations of sperm in usual BTS and in BTS-EDTA, whereas after insemination with semen in BTS and in BTS + BTS-Caf/Ca, the recruitment was significantly reduced (p<0.01) (Table 2). In the animals inseminated with regular semen or with semen plus BTS-Cafeïne/Ca, almost 50% of the total recruited PMN was found in the back flow. In contrast, in animals inseminated with semen in BTS-EDTA this proportion was only 20%.

**Table 2:**

| mean number of PMN recovered, ± s.e. | | |
|---|---|---|
| | Backflow | Back flow + Genital tract |
| regularBTS (n=6) | 2.16 (±0.70) | 4.44 (±1.19) |
| BTS-Caf/Ca (n=5) | 0.59 (±0.40) | 1.33 (±0.71) |
| BTS- EDTA (n=6) | 1.25 (±0.53) | 5.59 (±1.33) |

### Sperm recovery.

Like the number of recruited PMN, the total number of recovered sperm per sow varied between the individual animals. This variation was even more marked after insemination with semen in BTS-EDTA. No significant differences were found in the total number of recovered sperm between the sows inseminated with semen of boar A or boar B. Spermatozoa were found in all oviducts irrespective of the composition of the inseminate.

In all three treatment groups, the total number of sperm recovered at four hours after insemination (i.e. inside plus outside PMN, in the back flow plus the genital tract) was approximately 50 % of the number of sperm inseminated.(Table 3)

**Table 3:**

| Number* of recovered sperm. | | | |
|---|---|---|---|
| | Backflow | Genital tract | Total |
| regularBTS (n=6) | 43.1 (±8.9) | 5.2 (±1.9) | 48.7 (±7.3) |
| BTS-Caf/Ca (n=5) | 31.8 (±6.2) | 18.8 (±4.2) | 51.7 (±9.0) |
| BTS- EDTA (n=6) | 37.7 (±8.4) | 9.3 (±2.6) | 47.4 (±7.3) |

| | | | |
|---|---|---|---|
| Mean ± SE * expressed as a percentage of number of sperm in the inseminate). | | | |

The total number of sperm in the genital tract (i.e. within plus outside PMN) was significantly (p<0.01) higher in the BTS + BTS-Caf/Ca group when compared to the regular BTS group (table 3). Moreover, a significantly lower proportion of these spermatozoa were found inside PMN (table 4). The number of free, non-phagocytosed sperm in the uterus was clearly much higher (highly significant P<0.001) in the BTS + BTS-Caf/Ca group when compared to the regular BTS group (table 5). At four hours after insemination the number of sperm in the oviducts was not higher in the BTS + BTS-Caf/Ca group (table 5).

**Table 4:**

| Percentage of sperm found inside PMN at 4 hours after insemination. | | | |
|---|---|---|---|
| Inseminate | Back flow | Vagina/Cervix | Uterus |
| BTS | 21.5 (±5.8) | 73.5 (±6.7) | 66.5 (±10.0) |
| BTS +caffeine/Ca²⁺ | 15.1 (±6.1) | 48.5 (±8.6)* | 28.6 (±10.5)* |
| BTS+excess EDTA | 32.1 (±6.8) | 78.3 (±6.6) | 84.8 (± 7.5) |

| | | | |
|---|---|---|---|
| Mean (± SE) * Value differed significantly from that of the regular BTS group (p<0.05). | | | |

**Table 5:**

| Number* of free, non-phagocytosed sperm in uterus and oviducts PMN at 4 hours after insemination. | | |
|---|---|---|
| | Uterus | Oviducts |
| Reference | 1.07 (± 0.16) | 0.0071 (± 0.0016) |
| Caffeine/calcium | 14.95 (± 3.11) | 0.0040 (± 0.0014) |
| Excess EDTA | 1.38 (± 0.36) | 0.0026 (± 0.0010) |

| | | |
|---|---|---|
| Mean (± SE) * expressed as a percentage of number of sperm in the inseminate). | | |

As to semen back flow and sperm recovery in the uterus, the differences between the BTS-EDTA group and the regular BTS group were small and not significant. The sperm recovery in the oviducts was significantly lower in the BTS-EDTA group(p<0.05). Observations in the individual animals revealed some remarkable extremes. In two sows respectively 87% and 75% of the inseminated sperm were recovered in the back flow, whereas in two other animals this was only 4% and 3%. In only one of the two latter animals this reduced back flow coincided with a slightly higher number of non-phagocytized sperm in the uterus.

The results show that the use of caffeine in combination with Ca²⁺ significantly reduced PMN recruitment during the first four hours after insemination. Also, the use of caffeine/Ca²⁺ drastically enhanced the number of free, not-phagocytosed sperm that were still present in the uterus at four hours after insemination, indicating that phagocytosis of sperm had been seriously reduced. Wihout being bound thereto it is thought that the reduction of phagocytosis is due partly to an effect on the spermatozoa that results in a reduced rate of phagocytosis by PMN, as was found earlier in in vitro experiments, and partly on the reduced number of PMN present in the genital tract.

Both effects are due to the action of caffeine as an inhibitor of phosphodiesterase.

The in vitro phagocytosis experiments however, did show that phagocytosis of sperm by PMN was indeed decreased by addition of caffeine, especially when used in combination with Ca²⁺. This effect, however, was due to some action of the active compounds on the spermatozoa, and not to an effect on the PMN.

It has been shown that treatment of sperm for in vitro capacitation also reduces the rate of phagocytosis of sperm by PMN considerably. The effect of caffeine/Ca²⁺ on phagocytosis of sperm in vitro does not come down to a stimulation of capacitation. In at least some of our in vitro experiments in which we showed the effect of caffeine on phagocytosis of sperm, the sperm were not washed free of the seminal plasma. Seminal plasma is known to inhibit sperm phagocytosis. Furthermore, the effect of caffeine was seen without added calcium ions and in the presence of EDTA. This totally inhibits sperm capacitation (Harkema et al. 1998). On the other hand, it was noted that calcium ions augment the effect of caffeine.

In the present invention, the use of caffeine/Ca²⁺ did not result in a higher number of sperm present in the oviducts. The passage from the uterus to the oviducts , the so-called utero-tubal junction, seems to be designed to keep access of sperm to the oviducts at a very low level. This is suggested to be vital for the prevention of polyspermy. The sperm population in the oviducts, is reported to continue to grow during the first 24 hours after insemination due to continued passage of sperm from the uterus (Pursel, et al. 1978). Consequently, the higher number of sperm found to be present in the uterus after insemination with caffeine/Ca²⁺ is favourable for the chance of fertilisation, especially when the interval between insemination and ovulation is increased. Moreover, the fact that caffeine/Ca²⁺ decreases the elimination of sperm from the genital tract shoes that the same uterus sperm population as is achieved with 'normal' inseminations in regular BTS can be achieved with a substantial lower number of sperm in the inseminate when using BTS plus caffeine/Ca²⁺.

In in vitro experiments it was also found that another way to reduce phagocytosis of sperm in vitro was to lower the free Ca²⁺ concentration by EDTA. However, in the present in vivo study insemination with semen in BTS with excess sodium EDTA had no positive effects. Neither recruitment of PMN nor the extent of elimination of sperm from the genital tract was attenuated. It is suggested that as soon as the inseminate fluid containing the EDTA had been expelled and Ca²⁺ and other metal ions become available from uterine mucus, the PMN could still start with a rapid ingestion of sperm. Since the presence of EDTA would also have retarded the process of capacitation these spermatozoa would then be especially vulnerable for phagocytosis. Indeed, it turned out that the presence of extra EDTA in the inseminate had a negative effect on the number of sperm in the oviducts.

### Example III

### CAFFEINE PLUS CA2+ REDUCES UTERINE LEUKOCYTE RECRUITMENT AND SPERM PHAGOCYTOSIS AND IMPROVES FERTILITY IN PIG AI

Within hours after insemination the number of sperm in the female genital tract is reduced dramatically due to phagocytosis of sperm by uterine PolyMorphoNuclear leukocytes PMN) (Pursel, V.G., et al. (1978) Biol. Reprod. 19: 69-76), which are recruited in vast numbers to the lumen of the uterus shortly after AI. Caffeine (plus CaCl2) markedly reduces the rate of sperm phagocytosis in vitro. The effect of caffeine on PMN recruitment, numbers of not-phagocytosed sperm in the genital tract, the percentage of fertilised oocytes, and the number of accessory sperm is measured.

### MATERIALS AND METHODS

Series 1: Batches of sows (parity 3-12) received hCG at 96 hours after weaning. Sows showing oestrus were inseminated 26 hours after hCG with H33342 labelled semen with either 1 x 109 sperm in 80 ml normal BTS extender, or 1 x 109 sperm in 40 ml normal BTS followed by 40 ml of BTS minus EDTA and with 6 mM CaCl2 and 2,3 mM caffeine. Semen backflow was collected using stoma bags around the vulva. The animals were_slaughtered at four hours after insemination. Vagina, cervix and uterus were flushed repeatedly with PBS. Sperm and PMN were counted in the flushings and the backflow. The tractus parts, including the oviducts, were then homogenised to count (remaining) sperm.
Series 2: Batches of sows (parity 2-13) received hCG at 84 hours after weaning. The sows were inseminated at either 16 hours after hCG, on average 26 (range 21-28) hours before ovulation as observed by transrectal ultrasonography, or inseminated at 4 hours after ovulation. The sows were inseminated with 0.5 x 109 sperm in 40 ml normal BTS followed by 40 ml of either normal BTS or BTS minus EDTA and plus 6 mM CaCl2 and 2.3 mM caffeine. The sows were slaughtered at 115 (112-120) hours after ovulation. The embryos were flushed, examined morphologically, and the number of accessory sperm were counted.

### RESULTS AND DISCUSSION

The use of caffeine/CaCl2 significantly reduced PMN recruitment and resulted in significantly higher sperm number in the uterus (P<0.01) at 4 hr. after AI. Oviduct sperm number was not significantly different. Caffeine/CaCl2 resulted in a significantly higher number of accessory sperm in the sows inseminated 26 hours before ovulation. The % fertilised oocytes tended to be higher with caffeine (not significant).
The addition of Caffeine/CaCl2 reduces the rate at which sperm are eliminated from the genital tract. The improved fertility at 26 hours after AI shows that sperm number in the oviducts remain longer at a sufficient level, which could have consequences for field fertility in pig AI and/or could enable a reduction of sperm dosage.

| **Series 1** | | |
|---|---|---|
| **Number of sperm** (x 10⁶) | Control | Caffeine/Ca |
| | | |
| Inseminated | 1150 | 1080 |
| Backflow¹ | 495 ±75 | 313 ± 92 |
| Total in tractus¹ | 58 ± 14 | 216 ± 72 |
| Uterus² | 13 ± 1.1 | 162 ± 77 |
| Oviducts² | 0.08 ± 0.03 | 0.04 ± 0.01 |

| **Number of PMN (x 10**^{**6**}**)** | | |
|---|---|---|
| Total tract + backflow | 4458 ± 1029 | 1327 ± 593 |

| | | |
|---|---|---|
| ¹ including sperm inside PMN. | | |
| ² only free, not-phagocytosed sperm | | |

| **Series 2** | | | | |
|---|---|---|---|---|
| | AI ±26 hrs before ovulation | | AI ±4 hrs after ovulation | |
| | Control | Caffeine/Ca | Control | Caffeine/Ca |
| | n = 16 | n = 16 | n = 16 | n = 17 |
| % norm. Embryos¹ | 89.9 | 96.4 | 98.5 | 100 |
| # of accessory sperm² | 5.8^{a} | 16.2^{bc} | 44.6^{c} | 41.9^{c} |
| # of cells per embryo¹ | 39± 11 | 52± 26 | 35 ± 9 | 38 ±12 |

| | | | | |
|---|---|---|---|---|
| ¹ not significant. | | | | |
| ² a,b, c significantly different (P<0.001). | | | | |

### Example IV

Influence of treatment for in vitro capacitation of boar sperm on phagocytosis by PMN.

| Time (min) | % sperm phagocytosed | |
|---|---|---|
| | Non-capacitated | Capacitated |
| 0 | 0 ± 0 | 0 ± 0 |
| 15 | 61 ± 2.5 | 20 ± 2.0 |
| 30 | 70 ± 2.3 | 27 ± 2.3 |
| 45 | 73 ± 2.2 | 33 ± 2.6 |
| 60 | 77 ± 2.0 | 35 ± 2.5 |
| 75 | 78 ± 2.0 | 35 ± 2.5 |
| 90 | 81 ± 1.9 | 41 ± 2.8 |

% sperm phagocytosed by PMN in vitro, mean (n=3) ± s.e.

## Claims

1. A method for the artificial insemination of animals, which method comprises artificially inseminating the animal with sperm combined with a phosphodiesterase inhibitor or a functional equivalent thereof and optionally a soluble salt of an earth alkaline metal.

2. Method according to claim 1, wherein the soluble salt of an earth alkaline metal is a calcium salt.

3. Method according to claim 1 or 2, wherein caffeine is used as the phosphodiesterase inhibitor or functional equivalent thereof.

4. Method according to claims 1-3, wherein the phosphodiesterase inhibitor or functional equivalent thereof is used in an amount up to 10 mmol per litre inseminate.

5. Method according to claims 1-4, wherein the calcium salt is used in a concentration of up to 10 mmol added calcium per litre inseminate.

6. Method according to claims 1-5, wherein calcium chloride is used in an amount of 0.1 up to 8 mmol added calcium chloride per litre inseminate.

7. Method according to claims 1-6, wherein the animal is a domestic mammal or a bird.

8. Method according to claim 7, wherein the mammal is a pig.

9. Method according to claim 1, wherein the animal is inseminated with sperm followed by the introduction of a composition comprising a phosphodiesterase inhibitor or a functional equivalent thereof and a soluble salt of an earth alkaline metal.

10. Use of a phosphodiesterase inhibitor or a functional equivalent thereof in combination with a soluble salt of an earth alkaline metal in a method for artificial insemination of animals

11. Use of a phosphodiesterase inhibitor or functional equivalent thereof in combination with a soluble salt of an earth alkaline metal for elongating the functional life span of sperm.

12. Use of a phosphodiesterase inhibitor or a functional equivalent thereof in combination with a soluble salt of an earth alkaline metal in a composition which further comprises sperm for the reduction of the recruitment of polymorphonuclear neutrophils.

13. Use of a phosphodiesterase inhibitor or functional equivalent thereof in combination with a soluble salt of an earth alkaline metal in a composition further comprising sperm or the reduction of phagocytosis of sperm.

14. Inseminate comprising a phosphodiesterase inhibitor or functional equivalent thereof and a a soluble salt of an earth alkaline metal.

15. Inseminate according to claim 15, wherein the phosphodiesterase inhibitor or functional equivalent thereof is caffeine.
